# EUROPEAN PATENT SPECIFICATION

(11) **EP 2 295 033 B1**
(45) Date of publication and mention of the grant of the patent: **10.04.2019**
(21) Application number: 10189923.5
(22) Date of filing: 11.05.2007
(51) Int. Cl.: A61Q 19/00, A61K 8/81, A61Q 19/10, A61K 8/89, A61K 8/37, A61Q 1/06, A61Q 5/02, A61Q 5/06, A61Q 5/12, A61Q 15/00, A61Q 17/04, A61K 8/92, A61K 8/31, A61K 8/58

(54) **Cosmetic compositions containing side chain crystalline (scc) polymers**
Kosmetische Zusammensetzungen enthaltend seitenkettenkristallisierbare Polymere
Compositions de soins personnels comprenant des polymères fonctionnalisés (SCC)

(30) Priority: 11.05.2006 US 799616 P; 12.02.2007 US 900847 P; 11.05.2007 US 747261
(43) Date of publication of application: 16.03.2011
(62) Divisional of application: 07794820.6
(73) Proprietor: Evonik Degussa GmbH, 45128 Essen (DE)
(72) Inventor: Noor, Mussarat, Roselle Park, NJ 07204 (US); Lemma, Solomon, Orefield, PA 18069 (US)

(56) References cited:
- EP-A2- 1 466 579
- WO-A-01/19333
- US-A1- 2003 147 946
- US-A1- 2004 005 279
- US-A1- 2005 031 565
- US-A1- 2005 123 493
- US-A1- 2005 137 117
- US-A1- 2005 169 865
- US-A1- 2005 175 570
- US-A1- 2005 261 159

## Description

This Application claims the benefit of U.S. Provisional Application No. 60/799616, filed on May 11, 2006 and U.S. Provisional Application No. 60/900847, filed February 12, 2007.

### BACKGROUND OF THE INVENTION

### FIELD OF THE INVENTION

This invention relates to personal care or cosmetic compositions comprising functionalized polymers described in claim 1.

### INTRODUCTION TO THE INVENTION

U.S. Patent Nos. 4,057,622, 4,057,623, 4,057,624, 5,318,995, 5,519,063 and 5,736,125 disclose the possibility of thickening oil-containing compositions with certain polymers containing (a) lipophilic groups (e.g. in units derived from long chain n-alkyl acrylates) and (b) certain other groups, namely amide groups (in units derived from arylamide), pyrrolidone groups (in units derived from N-vinyl pyrrolidone), imidazole groups (in units derived from N-vinyl imidazole), carboxylic acid and carboxylic acid salt groups(e.g. in units derived from acrylic or Methacrylic acid), sulphonic acid groups, and sulphonic acid salt groups.

Side Chain Crystalline Polymers (SCC) are disclosed in Application Serial Nos. 11/199,049; and 11/199,508. Publications describing SCC polymers include U.S. Patent Nos. 4,830,855, 5,120,349, 5,156,911, 5,387,450, 5,412,035, 5,665,822, 5,783,302, 5,752,926, 5,807,291, 5,469,867, 5,826,584; 6,989,417 (Steven P. Bitler) and 7,101,928 (Steven P. Bitler).

### BRIEF SUMMARY OF THE INVENTION

In accordance with certain aspects of the present invention, a broad range of functionalized side chain crystalline (SCC) polymers can be used to thicken oils, provided that the SCC polymer will dissolve in the oil at a temperature above the crystalline melting point of the polymer (referred to herein as Tₚ) and can crystallize when the solution of the polymer in the oil is cooled to a temperature which is below Tₚ and at which the thickened oil composition is to be used. Without wishing to be bound by any theory or explanation, it is believed that the SCC polymer crystallizes into a network in which the polymer crystallites are connected to one another by semi-soluble chains.

In one aspect of the invention, the invention provides a personal care composition and method for making and using the composition wherein the SCC polymer has at least one functionality selected from the group consisting of hydroxyl. Such functionalized SCC polymers can be employed in a wide range of personal care compositions including skin care, body wash, shampoo, hair styling and hair treatments (e.g., hair molding cream, combing cream and pomade, hair sprays, hair colorant), sunscreen, lipstick, anti-perspirant , deodorant, shaving and after shaving products and among other personal care products. The amount of SCC polymer is sufficient to thicken, modify rheology, film form, enhance aesthetics, or improve sensory and feel, especially silicone, among other benefits.

A method is disclosed of making a composition, the method comprising
(i) dissolving the SCC polymer in the oil at a temperature above Tₚ, and
(ii) cooling the solution to crystallize the polymer in the oil.

In a further aspect of the invention, an advantage of using these SCC polymers as thickening agents in water-in-oil emulsions are that the need to use a surface active agent and any other water or oil phase thickener or rheology modifier other than SCC is reduced or removed. Thus the compositions can contain less than 5%, typically less than 2%, usually less than 1%, and in some cases about 0%, of surface active agents or other oil phase or water phase thickeners or rheology modifiers, the percentages being by weight based on the weight of the oil. This is particularly useful in cosmetic and personal care products, since it is conventional for such products to contain surface active agents (for example, perfluoroalkyl organic compounds), and surface active agents can cause an adverse reaction when they contact human skin.

### DETAILED DESCRIPTION OF THE INVENTION

In the Summary of the Invention above, and in the Detailed Description of the Invention, the Examples, and the Claims below, reference is made to particular aspects of the invention. It is to be understood that the disclosure of the invention in this specification includes all appropriate combinations of such particular features. For example, where a particular aspect or feature is disclosed in the context of a particular embodiment or a particular claim, that feature can also be used, to the extent appropriate, in the context of other particular embodiments and claims, and in the invention generally.

The invention is particularly useful for personal care compositions, for example cosmetics, toiletries, and cleansers, including but not limited to lipsticks, deodorant sticks, nail varnishes, creams, gels and oils, including sunscreen, hand protective products, night renewal products, body milks, creams and lotions, light facial products, protective day care products, liquid moisturizing emulsions and water-in-oil creams, as well as thickened oil products with water and products designed to assist in removing other cosmetic, makeup or personal care products. The invention is also useful in other contexts, for example in paints, film-forming compositions, inks, and compositions carrying active ingredients such as UV absorbers, fragrances, antimicrobial agents, germicides, antioxidants, preservatives, enzymes, nutrients, minerals and if desired, the foregoing can be supplied in a controlled-release format.

The instant invention can employ at least one of functionalized side chain crystalline polymers (FSCC) like Poly C12-22 Alkyl Acrylates/Hydroxyethylacrylate Copolymer (Intelimer® 1261, Intelimer® 1266). The following are examples of some of the benefits that can be obtained by using the FSCC polymers:
1. Ability to modify the rheology of cosmetic emulsion products or anhydrous products by thickening the oil phase,
2. Ability to modify the rheology of cosmetic emulsion products or anhydrous products by thickening the oil phase of silicone oil based formulations,
3. Compatibility in wide variety of cosmetic ingredients,
4. Usage in 100% solid form, or emulsified,
5. Improved water resistance and rub off resistance in cosmetic formulation for skin and hair,
6. Incorporation of active ingredients in the amorphous crystalline matrix structure and ability to release active ingredient in more controlled manner
7. Ability to enhance SPF of sunscreen formulations,
8. Ability to provide breathable film in cosmetic formulations on skin,
9. Film properties can be strengthened and converted into more occlusive (moisture retaining) film in combination of other film forming polymers soluble in oil or water phase along with these polymer
10. Ability to provide fragrance retention and volatile ingredient retention,
11. Ability to make lamellar gels more water resistant without increasing their viscosity,
12. Ability to formulate water-resistant emulsions based on alcohol and/or nonionic ethoxylated emulsifiers,
13. Sensory and texture benefits can be modified and enhanced by using different SCC polymer functionalities, among other benefits that would be understood by a person having ordinary skill in this art.

### Definitions and Abbreviations

In this specification, parts and percentages are by weight, except where otherwise noted. Temperatures are in °C. The onset-of-melting temperature, Tₒ, the peak melting temperature, Tₚ, and the heat of fusion, J/g, are determined using a differential scanning calorimeter (DSC) at a rate of temperature change of 10 °C./min, for example from -10 to 150 °C., and on the second heat cycle. Tₚ is the temperature at the peak of the DSC curve, and Tₒ is the temperature at the intersection of the baseline of the DSC peak and the onset line, the onset line being defined as the tangent to the steepest part of the DSC curve below Tₚ. The abbreviations Mn and Mw are used to denote number average and weight average molecular weight in daltons, respectively, measured in tetrahydrofuran using size exclusion chromatography, configured with a Wyatt laser light scattering detector. Bulk viscosities given in the Examples for the polymeric thickeners are in centipoise and were measured using a Brookfield LVT viscometer with an electrically thermostatted Thermosel heater, controlled for example to 95 °C., and small sample adapter using spindles 4 and 7. Wt% or %w/w refers to weight percent; and q.s. means to incorporate an amount of the indicated material sufficient to make the sum of all %w/w equal to 100

The abbreviation CxA is used to denote an n-alkyl acrylate in which the n-alkyl group contains x carbon atoms, the abbreviation Cx alkyl is used to denote an n-alkyl group which contains x carbon atoms, and the abbreviation CxM is used to denote an n-alkyl methacrylate in which the n-alkyl group contains x carbon atoms. Other abbreviations are given elsewhere in the specification.

### The Polymeric Thickeners

The FSCC polymers used as thickeners in the present invention can be homopolymers, or copolymers of two or more comonomers, including random copolymers, graft copolymers and block copolymers (including thermoplastic elastomers). Two or more SCC polymers can be used together. The number average molecular weight of the SCC polymer is generally from about 10,000 to about 1,500,000, normally 12,000 to 1,000,000. The molecular weight of an SCC polymer is relatively unimportant to its Tₚ, but is generally an important factor in determining the Tₚ of other polymers.

The SCC polymer usually melts over a relatively small temperature range. The closer Tₚ is to room temperature, in general the more rapid the transition. The SCC polymer normally has an onset of melting temperature, Tₒ, such that Tₚ - Tₒ is less than Tₚ^{0.7} , generally less than Tₚ^{0.6}, particularly less than 10 °C., especially less than 6 °C., Tₒ and Tₚ being in °C. The crystallinity of the SCC polymer is typically such that its heat of fusion is at least 20 J/g, particularly at least 40 J/g.

The SCC polymer may for example be derived from one or more acrylic, methacrylic, olefinic, epoxy, vinyl, ester-containing, amide-containing or ether-containing monomers. SCC polymers can comprise repeating units in which the side chains comprise linear polymethylene radicals containing 10 to 50, e.g. 16-50, especially 16 to 22, carbon atoms. Polymers containing such units can be prepared by polymerizing a monomer component comprising one or more corresponding linear aliphatic acrylates or methacrylates, or equivalent monomers such as acrylamides or methacrylamides. A number of such monomers are available commercially, either as individual monomers or as mixtures of identified monomers, for example C12A, C14A, C16A, C18A, C22A, a mixture of C18A, C20A and C22A, and a mixture of C26A to C40A. The polymers also contain units derived from one or more other comonomers containing as suitable functional group hydroxyl- groups. Such monomers include, for example, those listed below. In the list below, the term (meth)acrylate means that the compound may be either an acrylate or a methacrylate: N-(2-hydroxyethyl)acetamide; hydroxyalkyl (in particular, hydroxyethyl, hydroxypropyl, and hydroxybutyl) (meth)acrylates; 1-acryloxy-2-hydroxy-3-phenoxypropane.

When the SCC polymer comprises a graft or block copolymer, it can be prepared by copolymerizing a vinyl type macromonomer with other monomers, or by making an SCC polymer, and then reacting the functionalized polymer with the second block material, for example a urethane block, an epoxy block, a polyether block, e.g. a polyethyleneoxide, polypropyleneoxide or polytetramethyleneoxide block, a polysiloxane block, or a poly(alkyl or alkoxy)silane block, or it can be prepared by using any other suitable method of polymerization such as emulsion or suspension polymerization in the presence of surface active agents and/or colloids, or bulk polymerization or solution polymerization. Non-limiting examples of surface active agents comprise at least one member of cetearth, laureth, pareth, PEG and/or PPG or POE esters and ethers of sorbitan Oleate, octyl phenyl and/or ester of nonylphenyl and sulfosuccinate, sodium or Disodium salts like Disodium C-isodecylslfosuccinate, sodium isostearate, esters or ethers of of natural and synthetic oils and waxes like PEG and/or PPG hydrogenated caster oils, among others. Non-limiting examples of colloids comprise at least one member of stearyl alcohols; behenyl alcohols; modified and unmodified gums like cargeenans, celluloses, carbomers; PVP; Hydrogenated and hydrolyzed waxes and/or oils; among others.

The SCC polymer is used to thicken a water-in-oil emulsion, and it contains at least about 5%, usually at least 10%, of units derived from a monomer containing as a functional group at least one hydroxyl functional groups and may contain higher amounts (e.g. up to 25% or 30wt%), provided that the SCC polymer will at least partially dissolve in the oil.

In one aspect of the invention, the SCC polymer consists essentially of
(i) 70-99% by weight of repeating units derived from at least one n-alkyl acrylate or methacrylate ester in which the n-alkyl group contains 16 to 22 carbon atoms,
(ii) 15-50%, by weight of repeating units derived from at least one acrylate or methacrylate ester in which the ester group contains a hydroxyl-substituted alkyl group containing less than 12 carbon atoms, and
(iii) 0-30% by weight of repeating units derived from at least one acrylate or methacrylate ester in which the ester group contains an unsubstituted alkyl group containing less than 16 carbon atoms to lower the Tp.

Suitable polymers within the foregoing aspect can include SCC polymers consisting essentially of
(i) 70-99% by weight of the repeating units derived from at least one n-alkyl acrylate or methacrylate ester in which the n-alkyl group contains 16 to 22 carbon atoms, and
(ii) 15-50%, by weight of the repeating units derived from at least one acrylate or methacrylate ester in which the ester group contains a hydroxyethyl, hydroxypropyl, or hydroxybutyl group.

The molecular weight and other properties of the SCC polymer can be sufficiently controlled such that the polymer, after it has been dissolved in the oil, will crystallize in the oil when the heated mixture is cooled to the expected temperature of use, for example to a temperature 10-20 °C. below Tₚ, thus producing an opaque or clear mixture.

The Tₚ of the thickening polymer is normally 10-40 °C. above, particularly 10-30 °C. above, especially about 20 °C. above, the temperature at which the composition is to be used, which is generally 15-40 °C. It appears that the oil plasticizes the thickening polymer, so that its melting point in the composition is for example 5-20 °C. lower than Tₚ. It is therefore desirable that Tₚ is sufficiently above the temperature of use to ensure that the thickening polymer does not melt during use. Thus for compositions to be used at around 20-35 °C. the thickening polymer can have a Tₚ of above 40 °C, usually 40-70 °C. On the other hand, if the Tₚ of the thickening polymer is too far above the temperature of use, this can result in excessive crystallization and then precipitation of the polymer, thus reducing the thickening effect. It is useful, therefore, that Tₚ is not more than about 30 °C. above, usually not more than 20 °C. above, the temperature of use. Depending on the expected temperature of use, Tp may be from 0-150 °C, generally 10-100 °C., typically 40-70 °C. , particularly 43-55 °C.

The amount of the polymeric thickener used may vary with the application. It is unnecessary for the amount of the thickener to be more than 10% by weight based on the weight of the oil. Smaller amounts such as about 2 to about 7% based on the weight of the oil in compositions which are free of water, and about 0.5 to about 5% based on the weight of composition in water-in-oil emulsions, are often effective. In the invention, the amount of polymeric thickener is sufficient to form a water in oil emulsion, having enhanced rheology and sensory properties. These properties can provide a sunscreen skin care emulsion having enhanced film building properties upon the skin and in turn increase the SPF of the sunscreen.

### Oils

The functionalized polymeric thickeners are effective with a broad range of oils such as at least one member selected from the group consisting of esters (C12-15 Alkyl benzoate), triglyceride (Caprylic/Caprylate triglyceride) hydrocarbons (mineral oil, sunflower oil) natural oils (jojoba oil, safflower oil) castor oil, among others. Suitable oils are also disclosed, for example, at column 3, line 37, to column 4, line 4, of U.S. Patent No. 5,736,125; hereby incorporated by reference. For thickening silicone oils, it is useful to use an SCC polymer containing units derived from a monomer containing silicon, for example a block copolymer containing SCC blocks and polysiloxane blocks. SCC/polysiloxane polymers of this type are described for example in WO 93/07194 and WO 00/04787; hereby incorporated by reference. Non-limiting examples of silicone oils can comprise at least one of dimethicone, pdms, organo silicone oils, dimethicones and cyclomethiconesln one aspect of the invention, the SCC polymers are employed as rhelogy modifiers (e.g., thickener) for at least one of dimethicone, cyclomethicone, and other low viscosity silicone oils..

### Water-in-oil Emulsions

Water-in-oil emulsions are typically prepared by mixing together (1) a hot solution of the thickener in the oil and (2) the aqueous phase, the aqueous phase being at a temperature similar to the oil solution (e.g. not more than 10 °C. different); and then cooling the mixture while stirring. The ratio of the aqueous phase to the oil phase can be, for example, about 0.5:1 to about 9:1.

One aspect of the invention, relates to using at least one of SCC and functionalized SCC polymers to prepare a water-in-oil emulsion. When employing SCC and functionalized SCC, the ratio of SCC to functionalized SCC can range from about 0:1 to about 10:1 The following are non-limiting examples of cosmetic formulations comprising water-in-oil emulsions:
A. Water in Silicone Oil Emulsions Containing Functionalized SCC and SCC
   i) Skin Moisturizer
      Water - about 50 to about 90wt%
      Silicone - about 1 to about 10wt%
      Emulsifier- about 0.5 to about 5wt%
      Emollient - about 5 to about 20wt%
      Functionalized SCC and/or SCC - about 0.5 to about 3%
      Other Additives - about 0.1 to about 3wt%
   ii). Sunscreen
      Water - about 50 to about 90wt%
      Silicone - about 1 to about 10wt%
      Emulsifier - about 0.5 to about 5wt%
      Emollient - about 5 to about 20wt%
      Functionalized SCC and/or SCC - about 0.5 to about 3wt%
      Sunscreen Active - about 1 to about 25wt%
      Other Additives - about 0.1 to about 3wt%
   iii) APDO
      Water - about 50 to about 90wt%
      Silicone - about 1 to about 10wt%
      Emulsifier - about 0.5 to about 5wt%
      Emollient - about 1 to about 20wt%
      Functionalized SCC and/or SCC - about 0.5 to about 6wt%
      Antiperspirant active - about 1 to about 25wt%
      Other Additives - about 0.1 to about 5wt%
B. Water in oil Emulsion Containing Functionalized SCC
   i) Skin Moisturizer
      Water - about 50 to about 90wt%
      Emulsifier - about 0.5 to about 5wt%
      Emollient - about 5 to about 20wt%
      Functionalized SCC - about 0.5 to about 3wt%
      Other Additives - about 0.1 to about 3wt%
   ii). Sunscreen
      Water - about 50 to about 90wt%
      Emulsifier - about 0.5 to about 5wt%
      Emollient - about 5 to about 20wt%
      Functionalized SCC - about 0.5 to about 3wt%
      Sunscreen Active - about 1 to about 25wt%
      Other Additives - about 0.1 to about 3wt%

### Additives

If desired, one or more properties of a cosmetic composition can be controlled by adding a plasticizing compound to the composition. Examples of such compounds comprise at least one member selected from the group consisting of silicone based plasticiziers, natural or synthetic compounds (e.g., polysaccharides, natural or synthetic Gums, stabilizers, anionic and noionic associative thickener or rheology modifiers soluble in oil or water phase), other film forming polymers like polyurethanes, pyrolidines (e.g., polyvinylpyrolidine), among other compounds In one aspect of the invention, the additives comprise at least one member selected from the group consisting of preservatives, stabilizers (e.g., Xanthan Gum), humectant (e.g., at least one of Glycerine, MP Diol, Sorbitol, and Hexylene Glycol), antioxidant (e.g., Vitamins), rheology modifiers, fragrances, pigments, among other additives. In a further aspect of the invention, the additive can comprise at least one of surfactants and foam boosters. While any suitable surfactant and/or foam booster can be employed, examples of such comprise at least one member selected from the group consisting of sodium laureth sulfate, sodium lauryl sulfate, ammonium laureth sulfate, ammonium lauryl sulfate, cocamidopropyl betaine, among others. In a still further aspect of the invention, the additive can comprise at least one propellant and solvent such as at least one of isobutene, butane, dimethyl ether, ethanol, among others. The amount of additive typically ranges from about 0.1 to about 30 wt.% of the composition.

### Emulsifiers

One or more emulsifiers is incorporated with the inventive composition. While any suitable emulsifier can be employed, examples of suitable emulsifiers comprise at least one member selected from the group consisting of glyceryl stearate, PEG-150 distearate, dlyceryl dilaurate, PEG-20 stearate, PEG-150 distearate, cetearyl alcohol (and) ceteareth-20, PEG-30 Dipolyhydroxystearate, among other compounds capable of forming or stabilizing an emulsion. The amount of emulsifier can range from about 0.5 to about 6 wt.% of the composition.

### Emollients

If desired, one or more emollients can be incorporated within the inventive composition. While any suitable emollient can be employed, examples of suitable emollients comprise at least one member selected from the group consisting of esters (e.g., C12-15 alkyl benzoate) and triglycerides (e.g., Caprylic/caprylate triglyceride); hydrocarbon oils (e.g., mineral oil), natural oil (e.g., Jojoba oil, safflower oil), tridecyl trimellitate, sunflower oil, castor oil, among other compounds used to impart improved sensory or aesthetic properties of a personal care composition. The amount of emollient can range from about 1 to about 30 wt.% of the composition.

### Active Compounds

The inventive composition can be employed for delivering active compounds that interact with or protect skin or hair selected from the group consisting of zinc oxide, titanium dioxide, octinoxate, octocrylene, ethylhexyl Salicylate, oxybenzone.

Certain aspects of the instant invention are illustrated by the following non-limiting Examples. The procedures used in the Examples to compare the effectiveness of the polymeric thickeners were as follows. In Examples 1-6, the thickener, 5 parts, was dissolved in hydrogenated polyisobutylene (HPIB, light oil), 95 parts, with stirring at 120 °C. The resulting solution was placed in an incubator at 20 °C. for 16 hours. The viscosity of the cooled product in centipoise was determined using a Brookfield DV-I+ digital viscometer with CP-51 spindle using a sample adapter which was thermostatically controlled, for example, to 25 °C. The viscosities were measured after four minutes at a speed of 2.5 rpm, i.e. after 10 revolutions. In Examples 7-12, the oil (as identified in Table 2), 14 parts, was heated to 80 °C., and the thickener, 0.75 parts, was dissolved therein. Water, 35 parts, containing MgSO₄.H₂O, 0.25 part, was heated to 80 °C. The oil and the water, both at 80 °C, were mixed together, and then cooled to 25 °C. with continued stirring. The product, a milky white water-in-oil emulsion, was left overnight, and its viscosity at 25 °C. was then measured using a Brookfield cone and plate viscometer. The viscosity was measured after 0.5, 1, 2 and 4 minutes, to assess the effect of shear on the emulsion.

The remaining Examples illustrate formulations of compositions incorporating functionalized SCC and/or SCC polymers. These Examples illustrate personal care formulations comprising skin care creams, lotions, and sun screens, body cleansing compositions (e.g., shower mouse), hair care (e.g., hair styling and conditioning), among other formulations. Examples according to the invention are marked with §, non-invenitve ones with *

### EXAMPLES

### Examples 1-12

Polymers and copolymers were made using the ingredients and amounts thereof shown in the Table below, using the following generalized method. To a resin kettle equipped with overhead stirrer and condenser was added 20% of the monomers and chain transfer agents. The mixture in the resin kettle was heated to 110 °C., and oxygen was removed from the system through nitrogen purge for about 30 min followed by addition of 20% of the starting initiator charge. After allowing sufficient time for any initial exotherm to abate, the remaining monomers, chain transfer agents and starting initiator were pumped into the reaction vessel over 60-90 min. The polymer mixture was allowed to continue reacting for 60 min followed by addition of the chase initiator and reaction for 60 min. The mixture was put under reduced pressure for 60 min to remove volatile residuals. The resulting polymers were generally yellow to white solids. The molecular weight, Tₚ, and viscosity of each sample were measured. The effectiveness of the polymers as thickeners was measured as described above, and the results are shown in Tables 1 and 2 below.

The following abbreviations are used in the Tables. ME = mercaptoethanol; MA = methacrylic acid; DMAEA = N,N-dimethylaminoethyl acrylate; HEA = 2-hydroxyethyl acrylate; TAPO = t-amylperoxy 2-ethylhexanoate sold by Witco as Esperox 570P, 75% active in liquid; TBPB = t-butylperoxybenzoate sold by Witco as Esperox 10; Estol is propylene glycol dicaprylate/caprate sold by Uniqema under the tradename Estol 1526; Min'l is mineral oil; and opq = opaque in appearance.

**TABLE 1**

| Example | 1§ | 2* | 3* | 4* | 5* | 6* |
|---|---|---|---|---|---|---|
| C16A | | | | 95 | | |
| C18A | 80 | | 95 | | 100 | 85 |
| C22A | | 95 | | | | |
| HEA | 20 | | | | | |
| DMAEA | | | | | | 15 |
| MA | | 5 | 5 | 5 | | |
| ME | 0.34 | 0.17 | 0.17 | 0.17 | 0.17 | 0.1 |
| TAPO | 1.73 | 1.73 | 1.73 | 1.73 | 1.73 | 1.73 |
| TAPB | 0.67 | 0.67 | | | | |
| TBPB | | | 0.5 | 0.5 | 0.5 | 0.5 |
| Tp °C. | 48 | 67 | 47 | 39 | 50 | 45 |
| J/g | 56 | 99 | 57 | 64 | 73 | 60 |
| Mw | 236K | | 427K | 1,000K | 950K | |
| Mn | 52K | | 240K | 520K | 230K | |
| Bulk viscosity | 4,000 | 2,500 | 19,000 | 24,000 | 2,000 | 350 |
| Visc in HPIB | 12,600 opq | 164 Opq | 2600 opq | <50 clear | 5400 Opt | 6000 opq |

In Example 2, within the HPIB oil the polymer had a Tₚ above a desirable range, which resulted in excessive crystallinity and poor thickening under the test conditions. However, in other oils (e.g. isopropyl palmitate, or isopropyl myristate) sufficient plasticization of Tp for the polymer of Example 2 may still result in effective thickening. In Example 4, within the HPIB oil the polymer had a Tₚ below the desired range, and was ineffective as a thickener under the test conditions, because it did not crystallize on cooling. However, the polymer of Example 4 could still provide thickening or film forming benefits within an oil where little plasticization occurred (e.g. mineral oil, or ethanol).

**TABLE 2**

| Example | 7§ | 8§ | 9§ | 10* | 11* | 12* |
|---|---|---|---|---|---|---|
| C18A | 80 | 80 | 80 | 100 | 100 | 100 |
| HEA | 20 | 20 | 20 | | | |
| ME | 0.34 | 0.34 | 0.34 | 0.17 | 0.17 | 0.17 |
| TAPO | 1.73 | 1.73 | 1.73 | 1.73 | 1.73 | 1.73 |
| TAPB | 0.67 | 0.67 | 0.67 | | | |
| TBPB | | | | 0.5 | 0.5 | 0.5 |
| Tp °C. | 48 | 48 | 48 | 50 | 50 | 50 |
| J/g | 56 | 56 | 56 | 73 | 73 | 73 |
| Mw | 236K | 236K. | 236 K | 950 K. | 950 K. | 950 K. |
| Mn | 52K | 52 K. | 52 K. | 230 K. | 230 K. | 230 K. |
| Bulk viscosity | 4,000 | 4,000 | 4,000 | 2,000 | 2,000 | 2,000 |
| Oil | HPIB | Min'l | Estol | HPIB | Min'l | Estol |

| Viscosity after | | | | | | |
|---|---|---|---|---|---|---|
| 0.5 min | 35K | 37K | 45K | 29K | ** | ** |
| 1.0 min | 35K | 38K | 40K | 28K | | |
| 2.0 min | 41K | 38K | 34K | 25K | | |
| 4.0 min | 46K | 40K | 34K | 28K | | |

| | | | | | | |
|---|---|---|---|---|---|---|
| ** separated. | | | | | | |

### Example 13*

Water resistant sunscreen emulsion based on non-ionic emulsifiers is formulated using following ingredients and procedure:

| INGREDIENTS - | %W/W |
|---|---|
| PHASE A | |
| Deionized Water | q.s |
| Hexylene Glycol | 2.00 |
| Carbomer (Carbopol 980) | 0.20 |
| | |

| PHASE B | |
|---|---|
| Octinoxate | 7.50 |
| Oxibenzone | 3.00 |
| Ethylhexyl Salicylate | 3.00 |
| Ethylhexyl Palmitate | 6.00 |
| PEG-20 Stearate | 2.00 |
| Glyceryl Dilaureth | 3.00 |
| Myreth-3 myristate | 2.0 |
| | |

| PHASE C | |
|---|---|
| Deionized Water | 5.00 |
| Triethanolamine, 99% | 0.20 |
| | |

| PHASE D | |
|---|---|
| C8-22 AlkylAcrylates/Methacrylic Acid Crosspolymer | 1.5 |
| | |

| PHASE E | |
|---|---|
| Propylene Glycol (and) Diazolidinyl Urea (and) Iodopropynyl Butylcarbamate | 0.60 |
| Phenoxyethanol (and) Methylparaben (and) Isobutylparaben (and) Butylparaben | 0.50 |

### PROCEDURE

1. Combine Phase A at RT. Slowly sprinkle Carbopol into Phase A at RT with stirring. When uniform, begin heating to 70-75°C with stirring.
2. Combine Phase B; heat to 75-80°C; stir until uniform.
3. Slowly add Phase B to Phase A with homogenization at 70°C. When batch appears uniform; add Phase C with homogenization. When batch appears uniform, turn off heat. Switch to sweep at 60°C. Continue sweep throughout cool-down.
4. Add Phase D with stirring at 65°C.
5. Add Phase E with stirring at 45°C.

Result: Water resistance Sunscreen emulsion is intended as an all-over body/face and hand moisturizer and SPF. The SCC Polymer acts as a viscosity modifier, impartswater resistance and lubricity while yielding anon-oily feeling.
Example 16a*: The preparation of example 16 is repeated using Poly C10-30 alkyl acrylate
Example 16b§: The preparation of example 16 is repeated using Poly C12-22 Alkyl Acrylates/Hydroxyethylacrylate Copolymer
Example 16c*: The preparation of example 16 is repeated using C8-22 Alkyl Acrylates/Butyl Dimethicone Methacrylate Copolymer
Example 16d*: The preparation of example 16 is repeated using Poly Acrylic Acid/C8-22 Alkyl Acrylate Copolymer

### Example 14*

Water resistance sunscreen lotion is formulated using following ingredients and procedure:

| INGREDIENTS - | %W/W |
|---|---|
| PHASE A | |
| Deionized Water | q.s |
| Hexylene Glycol | 2.00 |
| Carbomer (Carbopol 980) | 0.20 |
| | |

| PHASE B | |
|---|---|
| Octinoxate | 7.50 |
| Oxibenzone | 3.00 |
| Ethylhexyl Salicylate | 3.00 |
| Ethylhexyl Palmitate | 6.00 |
| PEG-20 Stearate | 2.00 |
| Glyceryl Dilaureth | 3.00 |
| Myreth-3 myristate | 2.0 |
| C8-22 Alkyl Acrylates/Butyl Dimethicone Methacrylate Copolymer | 0.50 |
| | |

| PHASE C | |
|---|---|
| Deionized Water | 5.00 |
| Triethanolamine, 99% | 0.20 |
| | |

| PHASE D | |
|---|---|
| Propylene Glycol (and) Diazolidinyl Urea (and) Iodopropynyl Butylcarbamate | 0.60 |
| Phenoxyethanol (and) Methylparaben (and) Isobutylparaben (and) Butylparaben | 0.50 |

### PROCEDURE

1. Combine water and glycol of Phase A at RT. Slowly sprinkle Carbopol into Phase A at RT with stirring. When uniform, begin heating to 70-75°C with stirring.
2. Combine Phase B; heat to 75-80°C; stir until uniform.
3. Slowly add Phase B to Phase A with homogenization at 70°C. When batch appears uniform; add Phase C with homogenization. When batch appears uniform, turn off heat. Switch to sweep at 60°C. Continue sweep throughout cool-down.
4. Add Phase D with stirring at 45°C and stir to RT.

Results: Sunscreen lotion with velvety feel due to use of C8-22 Alkyl Acrylates/Butyl Dimethicone Methacrylate Copolymer.
Example 22a§: The preparation of example 22 is repeated using Poly C12-22 Alkyl Acrylates/Hydroxyethylacrylate Copolymer
Example 22b*: The preparation of example 22 is repeated using C8-22 AlkylAcrylates/Methacrylic Acid Crosspolymer
Example 22c*: The preparation of example 22 is repeated using Poly Acrylic Acid/C8-22 Alkyl Acrylate Copolymer

### Example 15§

Sunscreen cosmetic/personal care formulations were formulated using following ingredients and procedure:

### In-Vitro SPF 29

| Sunscreen | %w/w |
|---|---|
| INCI Name | |
| Phase A | |
| Octinoxate | 7.5 |
| Benzophenone-3 | 6.0 |
| Octyl Salicylate | 5.0 |
| Mineral Oil | 3.0 |
| C12-15 alkyl benzoate | 5.0 |
| C12-22 Alkyl Acrylates/ Hydroxyethylacrylate Copolymer | 3.0 |
| Steareth-2 | 2.5 |
| Steareth-21 | 2.5 |

| Phase B | |
|---|---|
| Xanthan Gum | 0.2 |
| DI Water | q.s. to 100 |
| MP Diol | 5.0 |

| Phase C | |
|---|---|
| Panthenol | 0.25 |

| Phase D | |
|---|---|
| Propylene Glycol (and) Diazolidinyl Urea(and) Methyl Paraben (and) Propyl Paraben | 1.00 |
| Total | 100.0 |

### Sunscreen Formulation:

### Ingredient

| Phase A | % w/w |
|---|---|
| DI Water | q.s to 100 |
| Hydroxy Propyl Methylcellulose | 0.1 |
| Xanthan Gum | 0.2 |

| Phase B | |
|---|---|
| Octinoxate | 7 |
| Oxibenzone | 6 |
| Octyl Salicylate | 5 |
| C12-15 Alkyl Benzoate | 5 |
| Potassium Cetyl Phosphate | 0.5 |
| Sorbitan Oleate | 0.1 |
| C12-22 Alkyl Acrylates/Hydroxyethylacrylate Copolymer | 2 |
| Caprylyl/Caprylic Triglyceride | 10 |

| Phase C | |
|---|---|
| Panthenol | 0.3 |

| Phase D | |
|---|---|
| Propylene Glycol (and) Diazolidinyl Urea(and) Methyl Paraben (and) Propyl Paraben | 1.0 |
| | |
| Vitamin E | 0.2 |
| Total | 100 |

### PROCEDURE:

1. Phase A; ingredients were combined at RT and heated to 75-80°C with stirring.
2. Phase B; ingredients were combined and heated to 75-80°C; stirred until uniform.
3. Phase B was added to Phase A with homogenization at 80°C. Batch was homogenized for 3 to 5 minutes. Phase C was added during homogenization process, switched to sweep at 60°C. Continue sweep throughout cool-down process.

Results: Cosmetic/personal care formulations having enhanced SPF and resistance to water rub off/removal:

## Claims

1. A personal care composition comprising
(1) water,
(2) at least one oil,
(3) at least one side chain crystalline polymer which contains hydroxyl groups wherein the amount of polymer is 10 % or less by weight based on the weight of the oil and sufficient to form an emulsion; and
(4) at least one emulsifier, and
(5) at least one active compound selected from the group consisting of zinc oxide, titanium dioxide, octinoxate, octocrylene, ethylhexyl salicylate and oxybenzone,
wherein the composition comprises a water-in-oil emulsion and
the side chain crystalline polymer contains at least 5% of units derived from a monomer containing a hydroxyl group.

2. The composition of Claim 1 wherein the oil comprises at least one silicone oil.

3. The composition of Claim 1 further comprising at least one surfactant, at least one propellant or at least one solvent.

4. The composition of Claim 1 wherein said emulsifier comprises at least one member selected from the group consisting of glyceryl stearate, PEG-150 distearate, glyceryl dilaurate, PEG-20 stearate, PEG-150 distearate, cetearyl alcohol (and) ceteareth-20, and PEG-30 Dipolyhydroxystearate.

5. The composition of Claim 1 wherein the composition comprises at least one member selected from the group consisting of cosmetics, color cosmetics, toiletries, cleansers, blush, lipsticks, antiperspirant deodorants, deodorants, nail varnishes, sunscreens, hand protective products, anti-aging or skin renewal products, night renewal products, body milks, facial products, protective day care products, moisturizer, shampoo, hair gel, mascara, and hair sprays.

6. The composition of Claim 1 wherein the amount of emulsifier is 0.5 to 6 weight percent of the composition.

7. The composition of any of Claims 1 to 6, wherein the composition further comprises at least one member selected from the group consisting of triglycerides, C12-C15 alkyl benzoates, mineral oil and natural oils.

8. The composition of Claim 2 wherein the silicone oil comprises at least one of dimethicone and cyclomethicone.

9. The composition of Claim 7 wherein said member comprises triglycerides or wherein said member comprises one of mineral oil and natural oil.

10. A method for preparing a personal care composition comprising combining the ingredients of Claim 1.

## Patentansprüche

1. Körperpflegezusammensetzung, umfassend:
(1) Wasser,
(2) mindestens ein Öl;
(3) mindestens ein seitenkettenkristallisierbares Polymer, das Hydroxylgruppen enthält, wobei die Menge des Polymers 10 Gew.-% oder weniger beträgt, bezogen auf das Gewicht des Öls, und ausreicht, um eine Emulsion zu bilden; und
(4) mindestens einen Emulgator, und
(5) mindestens eine Wirkverbindung ausgewählt aus der Gruppe bestehend aus Zinkoxid, Titandioxid, Octinoxat, Octocrylen, Ethylhexylsalicylat und Oxybenzon,
wobei die Zusammensetzung eine Wasser-in-Öl-Emulsion umfasst, und
das seitenkettenkristallisierbare Polymer mindestens 5 % Einheiten enthält, die von einem Monomer abgeleitet sind, das eine Hydroxylgruppe enthält.

2. Zusammensetzung nach Anspruch 1, wobei das Öl mindestens ein Silikonöl umfasst.

3. Zusammensetzung nach Anspruch 1, ferner umfassend mindestens ein oberflächenaktives Mittel, mindestens ein Treibmittel oder mindestens ein Lösungsmittel.

4. Zusammensetzung nach Anspruch 1, wobei der Emulgator mindestens ein Element ausgewählt aus der Gruppe bestehend aus Glycerylstearat, PEG-150-distearat, Glyceryldilaurat, PEG-20-Stearat, PEG-150-Distearat, Cetearylalkohol (und) Ceteareth-20, und PEG-30-Dipolyhydroxystearat umfasst.

5. Zusammensetzung nach Anspruch 1, wobei die Zusammensetzung mindestens ein Element ausgewählt aus der Gruppe bestehend aus Kosmetika, Farbkosmetika, Toilettenartikeln, Reinigungsprodukten, Rouge, Lippenstiften, Antiperspirantdeodorantien, Deodorantien, Nagellacken, Sonnenschutzprodukten, Handschutzprodukten, Anti-Aging- oder Hauterneuerungsprodukten, Erneuerungsprodukten zur nächtlichen Anwendung, Körpermilchen, Produkten zur Gesichtspflege, schützenden Tagespflegeprodukten, Feuchtigkeitsprodukten, Shampoo, Haargel, Mascara und Haarsprays umfasst.

6. Zusammensetzung nach Anspruch 1, wobei die Menge des Emulgators 0,5 bis 6 Gewichtsprozent der Zusammensetzung beträgt.

7. Zusammensetzung nach einem der Ansprüche 1 bis 6, wobei die Zusammensetzung ferner mindestens ein Element ausgewählt aus der Gruppe bestehend aus Triglyceriden, C₁₂-C₁₅-Alkylbenzoaten, Mineralöl und natürlichen Ölen umfasst.

8. Zusammensetzung nach Anspruch 2, wobei das Silikonöl mindestens eines von Dimethicon und Cyclomethicon umfasst.

9. Zusammensetzung nach Anspruch 7, wobei das Element Triglyceride umfasst, oder wobei das Element eines von Mineralöl und natürlichem Öl umfasst.

10. Verfahren zur Herstellung einer Körperpflegezusammensetzung, umfassend Kombinieren der Bestandteile von Anspruch 1.

## Revendications

1. Composition de soin personnel, comprenant
(1) de l'eau,
(2) au moins une huile,
(3) au moins un polymère cristallin à chaîne latérale qui contient des groupements hydroxyle, où la quantité de polymère est de 10% ou moins en poids sur la base du poids de l'huile et suffisante pour former une émulsion, et
(4) au moins un émulsifiant, et
(5) au moins un composé actif choisi dans le groupe constitué par l'oxyde de zinc, le dioxyde de titane, l'octinoxate, l'octocrylène, le salicylate d'éthylhexyle et l'oxybenzone,
où la composition comprend une émulsion eau-dans-huile et
le polymère cristallin à chaîne latérale contient au moins 5% de motifs dérivés d'un monomère contenant un groupement hydroxyle.

2. Composition selon la revendication 1, où l'huile comprend au moins une huile de silicone.

3. Composition selon la revendication 1, comprenant en outre au moins un agent tensioactif, au moins un propulseur ou au moins un solvant.

4. Composition selon la revendication 1, où ledit émulsifiant comprend au moins un élément choisi dans le groupe constitué par le stéarate de glycéryle, le distéarate de PEG-150, le dilaurate de glycéryle, le stéarate de PEG-20, le distéarate de PEG-150, l'alcool cétéarylique (et) cétéareth-20, et le dipolyhydroxy-stéarate de PEG-30.

5. Composition selon la revendication 1, où la composition comprend au moins un élément choisi dans le groupe constitué par les cosmétiques, les cosmétiques colorés, les produits de toilette, les nettoyants, les fards à joues, les rouges à lèvres, les déodorants antitranspirants, les déodorants, les vernis à ongles, les écrans solaires, les produits de protection des mains, les produits antivieillissement ou de renouvellement cutané, les produits de renouvellement nocturne, les laits corporels, les produits pour le visage, les produits protecteurs de soin journalier, les hydratants, les shampooings, les gels coiffants, les mascaras, et les laques.

6. Composition selon la revendication 1, où la quantité d'émulsifiant va de 0,5 à 6% en poids de la composition.

7. Composition selon l'une quelconque des revendications 1 à 6, où la composition comprend en outre au moins un élément choisi dans le groupe constitué par les triglycérides, les benzoates de C₁₂-C₁₅-alkyle, une huile minérale et les huiles naturelles.

8. Composition selon la revendication 2, où l'huile de silicone comprend au moins une parmi la diméthicone et le cyclométhicone.

9. Composition selon la revendication 7, où ledit élément comprend des triglycérides ou où ledit élément comprend l'une parmi une huile minérale et une huile naturelle.

10. Méthode de préparation d'une composition de soin personnel, comprenant la combinaison des ingrédients selon la revendication 1.
